# FASCICULE DE BREVET EUROPEEN

(11) **EP 0 632 269 B1**
(45) Date de publication et mention de la délivrance du brevet: **14.07.1999**
(21) Numéro de dépôt: 94401429.9
(22) Date de dépôt: 24.06.1994
(51) Int. Cl.: G01N 33/544, G01N 33/566, G01N 33/547

(54) **Dispositif pour la capture de molécules cibles, et procédé de capture utilisant ledit dispositif**
Vorrichtung zum Auffangen von zielmolekülen und diese Vorrichtung benutzendes Auffangverfahren
Device for capturing target molecules and capturing procedure using the device

(30) Priorité: 25.06.1993 FR 9307797
(43) Date de publication de la demande: 04.01.1995
(73) Titulaire: BIO MERIEUX, 69280 Marcy l'Etoile (FR)
(72) Inventeur: Mabilat, Claude, F-69100 Villeurbanne (FR); Cros, Philippe, F-69005 Lyon (FR); Mandrand, Bernard, F-69100 Villeurbanne (FR); Charles, Marie-Hélène, F-69420 Condrieu (FR); Erout, Marie-Noelle, F-69110 Sainte Foy Les Lyon (FR); Pichot, Christian, F-69960 Corbas (FR)
(74) Mandataire: Tonnellier, Jean-Claude

(56) Documents cités:
- EP-A- 0 265 154
- EP-A- 0 524 864
- WO-A-90/13815
- WO-A-91/08307
- WO-A-91/19812
- US-A- 5 013 669

## Description

La présente invention a pour objet un dispositif pour améliorer la capture de molécules cibles et un procédé de capture utilisant ledit dispositif.

Il est de pratique courante d'utiliser des supports sur lesquels sont immobilisés des ligands, par exemple des protéines, haptènes, peptides, polypeptides, anticorps ou polynucléotides pour capturer des molécules cibles (molécules biologiques ou analogues), dans le but de les détecter et/ou de les doser, notamment dans la réalisation d'essais de diagnostic.

Un des soucis majeurs dans la réalisation de tels essais est d'améliorer la capture des molécules pour augmenter la sensibilité des tests.

A cet égard, la demande de brevet WO 91/08307 décrit un système dans lequel un premier oligonucléotide et une sonde de capture sont fixés de façon covalente sur un polymère soluble dans l'eau et le conjugué ainsi formé est, après capture de la cible, immobilisé sur un support solide par combinaison avec un second oligonucléotide qui est complémentaire du premier, et qui a été au préalable fixé, par covalence, sur le support solide.

Le brevet US-5013669 décrit un dispositif de capture comprenant un support solide microporeux et un conjugué polymère/ligand dans lequel le polymère existe sous une forme soluble dans l'eau et aussi sous une forme réticulée insoluble dans l'eau. Le conjugué est déposé sur le support solide, puis le polymère est réticulé et transformé en un agrégat formant une phase solide tridimensionnelle.

La Demanderesse a maintenant trouvé un nouveau dispositif et un procédé d'utilisation de ce dispositif pour améliorer la capture de molécules cibles qui simplifient avantageusement le système décrit dans la demande de brevet précitée.

Selon l'invention, le dispositif comprend un support solide et un conjugué immobilisé sur ledit support solide, ledit conjugué résultant du couplage covalent d'un copolymère avec une pluralité de molécules d'un ligand (au moins 2 molécules de ligand), ledit conjugué étant immobilisé directement sur le support solide par adsorption passive. Bien entendu, ni le ligand ni le polymère ne sont susceptibles de réagir sur le support solide avec formation d'une liaison covalente.

L'invention a donc pour objet un dispositif de capture d'une molécule cible dans le but de la détecter et/ou de la doser, ledit dispositif étant tel que défini dans la revendication 1.

Selon un premier mode de réalisation, le ligand est spécifique de la molécule cible. Dans ce mode de réalisation, le ligand est notamment choisi pour être susceptible de former un complexe de capture ligand/molécule cible. A titre d'exemple le complexe peut notamment être représenté par tout couple antigène/anticorps, anticorps/haptène, hormone/récepteur, chélatant/molécule chélatée, les hybrides polynucléotide/polynucléotide, polynucléotide/acide nucléique, ou analogues.

Le ligand est par exemple un polynucléotide ayant une séquence nucléotidique d'environ 5 à 100 nucléotides et suffisamment complémentaire de la séquence nucléotidique du fragment d'acide nucléique ou du polynucléotide à capturer pour permettre l'hybridation desdites séquences dans des conditions déterminées.

Dans un autre mode de réalisation, le ligand est capable de former un complexe avec un réactif bifonctionnel comprenant un groupement "anti-ligand", responsable de la formation du complexe avec le ligand, et dans lequel ledit groupement anti-ligand est lié, notamment par covalence, de façon connue, à un groupement partenaire de la cible. Le partenaire de la cible est un groupement capable de se lier avec la cible (en formant un complexe cible/partenaire) et est donc capable de capturer la cible, dans les conditions de l'essai, par établissement d'une liaison suffisamment forte pour assurer l'interaction cible-partenaire, par exemple par covalence et/ou par interactions ioniques et/ou par liaisons hydrogène et/ou par liaisons hydrophobes. Le complexe ligand/anti-ligand peut être dans ce cas tout couple mentionné ci-dessus pour le premier mode de réalisation, ou encore un complexe biotine/streptavidine, lectine/sucre ou analogues. En particulier le complexe ligand/anti ligand est un hybride polynucléotide/polynucléotide. Le complexe partenaire/cible est du même type que le complexe ligand/cible mentionné ci-dessus pour le premier mode de réalisation. Dans ce deuxième mode de réalisation, le système formé par le ligand fixé sur le support solide et par le réactif bifonctionnel permet d'immobiliser, par un phénomène de double capture, toute cible complémentaire du groupement partenaire choisi pour la réalisation du réactif bifonctionnel, et ce système constitue donc un dispositif universel de capture, c'est-à-dire utilisable quelle que soit la nature de la molécule cible, grâce à l'utilisation d'un réactif bifonctionnel comprenant un groupement partenaire approprié, comme expliqué ci-dessus.

Le copolymère utilisable dans le dispositif selon l'invention n'est pas nécessairement soluble dans les solvants aqueux ou organiques compatibles avec le support, mais le conjugué doit l'être. Ce polymère est de préférence un copolymère ayant une masse moléculaire généralement comprise entre 1000 et 500000, par exemple entre 10000 et 250000. Ledit copolymère peut être un copolymère statistique, alterné, greffé ou séquencé. On utilise en particulier des copolymères de N-vinyl pyrrolidone, de préférence des bipolymères.

Le copolymère provient de la copolymérisation d'un monomère de N-vinylpyrrolidone et d'un deuxième monomère approprié pour permettre l'établissement d'un couplage covalent entre le ligand et le copolymère. Par exemple, le deuxième monomère peut porter une fonction réactive telle que aldéhyde, époxy, halogénoalkyle, amine primaire, thiol, maléimide ou ester, (de préférence une fonction ester de N-hydroxysuccinimide), ou une fonction activable telle qu'une fonction carboxyle (activable notamment par formation d'un ester d'hydroxysuccinimide) ou qu'une fonction hydroxyle (activable notamment par le bromure de cyanogène). En particulier, les deux monomères sont la N-vinylpyrrolidone et le N-acryloxysuccinimide. Le copolymère comprend notamment de 25 à 70 %, en motifs, de motifs dérivés de la N-vinylpyrrolidone.

L'invention a également pour objet un procédé pour améliorer la capture de molécules biologiques, dans lequel la molécule cible, en solution dans un véhicule liquide, est mise en contact avec un dispositif tel que défini précédemment dans des conditions (connues) permettant de capturer la cible par formation d'un complexe de capture de type ligand/anti-ligand. Dans le premier type de réalisation mentionné ci-dessus, l'anti-ligand est la molécule cible. Dans le second mode de réalisation, l'anti-ligand est lui-même lié à un partenaire de la cible, tel qu'un anticorps (la cible est alors un antigène ou un haptène), un antigène, une protéine, un peptide ou un haptène (la cible est alors un anticorps), un polynucléotide (la cible contient alors un polynucléotide complémentaire) etc.

Le support et le procédé de l'invention sont utilisables notamment dans des tests pour la mise en évidence et la quantification d'une séquence nucléotidique dans un échantillon par exemple par des techniques sandwich ou "Reverse Dot". Ils sont également utilisables dans des immunoessais (selon la technique directe, sandwich, ou par compétition) pour capturer une molécule biologique.

On utilise des copolymères contenant deux motifs monomères différents dont l'un dérive de la N-vinylpyrrolidone. Ces copolymères sont appelés bipolymères dans la description de la présente demande.

Ces différents bipolymères peuvent être obtenus par voie radicalaire, par voie ionique, ou par réaction de transfert de groupe.

Le terme "polynucléotide" tel qu'utilisé dans la présente demande désigne un enchaînement d'au moins 5 désoxyribonucléotides ou ribonucléotides comprenant éventuellement au moins un nucléotide modifié, par exemple au moins un nucléotide comportant une base modifiée telle que l'inosine, la méthyl-5-désoxycytidine, la diméthylamino-5-désoxyuridine, la désoxyuridine, la diamino-2,6-purine, la bromo-5-desoxyuridine ou toute autre base modifiée permettant l'hybridation. Ce polynucléotide peut aussi être modifié au niveau de la liaison internucléotidique (comme par exemple les liaisons phosphorothioate, H-phosphonate, alkyl-phosphonate), au niveau du squelette comme par exemple les alpha-oligonucléotides (FR 2 607 507) ou les PNA (M. Egholm et al., J. Am. Chem. Soc., (1992), 114, 1895-1897). Chacune de ces modifications peut être prise en combinaison.

Le terme "support solide" tel qu'utilisé ici inclut tous les matériaux connu sur lesquels peut être immobilisé un polynucléotide et qui sont utilisés comme supports dans des tests diagnostiques, en chromatographie d'affinité et dans des processus de séparation. Des matériaux naturels, de synthèse, modifiés ou non chimiquement peuvent être utilisés comme support solide, notamment les polysaccharides tels que les matériaux de cellulose, par exemple du papier, des dérivés de cellulose tels que l'acétate de cellulose et la nitrocellulose, du dextran ; des polymères tels que polychlorure de vinyle, polyéthylène, polystyrène, polyacrylate, polyamide, ou copolymères à base de monomères vinyl aromatiques, esters d'acides carboxyliques insaturés ,chlorure de vinylidène, diènes ou composés présentant des fonctions nitrile (acrylonitrile) ; des copolymères chlorure de vinyle /propylène, ou chlorure de vinyle /acétate de vinyle ; des copolymères à base de styrène ou de dérivés substitués du styrène ; des fibres naturelles telles que le coton et des fibres synthétiques telles que le nylon ; des matériaux inorganiques tels que la silice, le verre, la céramique, le quartz ; des latex, c'est-à-dire des dispersions aqueuses colloïdales de polymère insoluble dans l'eau ; des particules magnétiques ; des dérivés métalliques.

De préférence, le support solide utilisé dans la présente invention est un polystyrène, un copolymère butadiène-styrène ou un copolymère butadiène-styrène en mélange avec un ou plusieurs polymères ou copolymères choisis parmi les copolymères styrène-acrylonitrile ou styrène-méthacrylate de méthyle, les polypropylènes, les polycarbonates ou analogues. Avantageusement, le support de la présente invention est un polystyrène ou un copolymère à base de styrène comprenant entre environ 10 et 90 % en poids de motifs dérivés du styrène.

Le support solide selon l'invention peut être, sans limitation, sous la forme d'une plaque de microtitration, d'une feuille, d'un cône, d'un tube, d'un puits, de billes, de particules ou analogues.

On entend par "fixation passive" une fixation due à des forces autres que des forces de liaison covalente.

Le terme "molécule biologique" ou "cible" tel qu'utilisé dans la présente invention inclut les acides nucléiques tels qu'ADN ou ARN ou leurs fragments, les antigènes, les haptènes, les peptides, les polypeptides, les anticorps, etc.

Le terme "anticorps" inclut également les fragments d'anticorps, et des anticorps obtenus par modification ou recombinaison génétique.

Le terme "haptène" désigne une molécule de taille insuffisante pour être immunogène, mais qui, par couplage avec une protéine par exemple, permet par immunisation d'animaux l'obtention d'anticorps reconnaissant ladite molécule.

La formation d'un conjugué résultant du couplage covalent d'un ligand, par exemple un polynucléotide à un copolymère peut être effectuée selon les méthodes dites directes ou indirectes connues.

Par exemple, dans le cas d'un polynucléotide, selon la méthode directe, on synthétise un polynucléotide ayant une fonction réactive sur un site quelconque de la chaîne nucléotidique comme par exemple, l'extrémité 5' ou l'extrémité 3', ou sur une base ou sur un phosphate internucléotidique, ou sur la position 2' d'un sucre. Le polynucléotide est ensuite couplé à un polymère, préparé au préalable et comportant une fonction réactive complémentaire de la précédente, c'est-à-dire permettant la formation d'une liaison covalente par réaction entre les deux fonctions réactives complémentaires, l'une portée par le polynucléotide et l'autre par le polymère. Par exemple, de façon connue, on peut coupler des amines primaires avec un acide carboxylique activé ou un aldéhyde ou bien une fonction thiol avec un halogénoalkyle. De préférence, la fonction réactive du polynucléotide pour le couplage sur le polymère est à l'extrémité 5' ou 3'.

Dans la méthode de couplage indirecte, le polynucléotide et le polymère sont chacun porteurs d'une fonction réactive, ces fonctions réactives pouvant être identiques ou différentes l'une de l'autre, ces deux fonctions n'étant pas complémentaires mais étant capables de réagir avec un agent intermédiaire de couplage qui est un réactif bifonctionnel (homobifonctionnel si les deux fonctions sont identiques ou hétérobifonctionnel si les deux fonctions sont différentes). Parmi les agents de couplage homobifonctionnels, on peut citer le DITC (phénylène-1,4-diisothiocyanate), le DSS (disuccinimidylsubérate) ou analogues. Parmi les agents de couplage hétérobifonctionnels, on peut citer le SMCC (succinimidyl-4-(N-maléimidométhyl) cyclohexane-1-carboxylate), ou le SMPB (succinimidyl-4-(p-maléimido phényl) butyrate), capables de réagir d'une part avec une amine primaire et d'autre part avec un thiol.

Après le couplage du ligand avec le copolymère, l'excès éventuel de fonctions réactives du copolymère est neutralisé de façon connue en soi. Par exemple, les groupements aldéhyde en excès peuvent être neutralisés par une amine primaire telle que l'éthanolamine, les groupements maléimide ou halogénoalkyle peuvent être neutralisés par un thiol (tel que thioéthanolamine ou dithiothréitol), etc.

L'invention a également pour objet un procédé pour améliorer la capture de molécules cibles, dans lequel la molécule cible en solution dans un véhicule liquide est mise en contact avec un dispositif tel que défini précédemment (c'est à dire le support solide auquel est fixé le conjugué polymère/ligand et, dans le cas du deuxième mode de réalisation, le support solide auquel est fixé le conjugué polymère/ligand et le réactif bifonctionnel anti-ligand/partenaire), dans des conditions permettant la formation d'un complexe de type ligand/antiligand ou la formation d'un complexe ligand/anti-ligand et cible/partenaire. Les conditions de formation de tels complexes sont connues en soi.

Les exemples suivants illustrent l'invention.

### EXEMPLE 1 : Synthèse et caractérisation des bipolymères :

Le protocole, qui suit, constitue le mode opératoire général des réactions de copolymérisation.

Le tableau 1 résumera, par la suite, les conditions particulières de cette synthèse.
- Dans un ballon tricol de 100 ml purgé à l'azote, sont mélangés 50 ml de diméthylformamide anhydre (ALDRICH ; référence 22705-6), une quantité x exprimée en grammes (g) de N-vinylpyrrolidone (ALDRICH; référence V 340-9) fraîchement distillée, ainsi qu'une quantité y (en grammes) de N-acryloxysuccinimide (KODAK; référence 110 1690).
- Le milieu réactionnel porté à une température de 60°C, est agité pendant 2 heures, sous bullage régulier d'azote, afin d'éliminer toute trace d'oxygène.
- z g d'acide 4,4' Azobis (4-cyano pentanoïque) (FLUKA ; référence 11 590) sont dissous dans 1 ml de diméthylformamide anhydre. Ce composé constitue l'amorceur de la réaction de polymérisation radicalaire.
- Après un bullage d'azote pendant 15 minutes, la solution d'amorceur est ajoutée rapidement au milieu réactionnel. Ceci constitue le temps zéro de la polymérisation.
- La réaction est conduite à 60°C, sous agitation, sous bullage lent et régulier d'azote.
- Des prélèvements sont effectués toutes les 3 minutes.

Pour stopper la réaction, sont ajoutés quelques grains d'un inhibiteur de polymérisation : l'hydroquinone (JANSSEN; référence 123-31-9). Les prélèvements sont ensuite placés dans la glace.
- Chacun de ces prélèvements est ensuite analysé en chromatographie en phase gazeuse (chromatographe DI 200, intégrateur ENICA 21, de la société DELSI INSTRUMENTS), grâce à une colonne SE 30 (méthylsilicone adsorbé sur Chromosorb 10 %). Ceci permet le suivi cinétique de la réaction de polymérisation, c'est à dire la connaissance, à tout instant, de la quantité de monomères résiduels, donc du taux de conversion (relatif à chaque monomère et global).

Conditions d'analyse :
Pression Air = Pression H2 = 1 bar (10⁵ Pa).
Pression N₂ = 0,8 bar.
Température du four = 175°C.
Température de l'injecteur = Température du détecteur = 300°C.

- Après un temps de réaction noté t et exprimé en heures, le milieu réactionnel est versé dans une ampoule à décanter, puis ajouté goutte à goutte dans un bécher contenant un grand excès d'éther éthylique (SDS, référence 440516) sous forte agitation.
- Le polymère, formé pendant la réaction, précipite sous la forme de cristaux blancs assez fins.

Quand tout le milieu réactionnel a été précipité, la solution d'éther éthylique est filtré sur fritté n° 4. Le polymère est lavé à l'aide d'un grand excès d'éther éthylique puis séché pendant plusieurs heures dans une étuve à vide, à température ambiante.
- Pour purifier le polymère (élimination des monomères résiduels ainsi que des faibles masses molaires), celui-ci est dissous dans le N-N diméthylformamide (DMF) puis reprécipité dans l'éther éthylique.
- Les bipolymères ainsi obtenus sont conservés sous atmosphère inerte, à l'abri de l'humidité. Ils sont constitués de motifs :

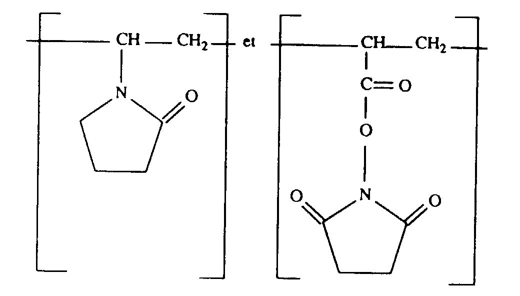

Le tableau 1 ci-après résume les conditions particulières à chaque réaction de polymérisation.

**TABLEAU 1**

| Dénomination des Bipolymères | x NVP (grammes) | y NAS (grammes) | C(*) moles/l | z AZCB (gramme) | T (heures) | R (**) (%) |
|---|---|---|---|---|---|---|
| COPO1 | 2,22 | 0,84 | 0,5 | 0,074 | 20 | 55 |
| COPO2 | 2,78 | 4,22 | 1,0 | 0,148 | 20 | 90 |
| COPO3 | 1,14 | 2,54 | 0,5 | 0,074 | 20 | 90 |
| COPO4 | 0,23 | 0,50 | 0,1 | 0,015 | 20 | 90 |
| COPO5 | 2,28 | 5,08 | 1,0 | 0,148 | 20 | 90 |

| | | | | | | |
|---|---|---|---|---|---|---|
| (*) C représente la concentration des monomères en moles/litre, en début de réaction. | | | | | | |
| (**) R représente la conversion, exprimée en pourcentage massique, des 2 monomères de départ. | | | | | | |

Abréviations utilisées dans le tableau 1 :
NVP = N-vinyl pyrrolidone,
NAS = N-acryloxysuccinimide,
AZCB = acide azobis 4-4' (4-cyanopentanoique).

Les caractéristiques des bipolymères utilisés dans la présente invention sont résumées dans le tableau 2.

**TABLEAU 2**

| Masse moléculaire (g/mole) (*) | | % Molaire de NAS dans le bipolymère final (**) |
|---|---|---|
| COPO1 | 60.000 (10 %) | 39 |
| COPO2 | 160.000 (2 %) | 57 |
| COPO3 | 70.000 (2 %) | 62 |
| COPO4 | 19.500 (2 %) | 59 |
| COPO5 | 160.000 (2 %) | 61 |

| | | |
|---|---|---|
| (*) Les masses moléculaires ont été déterminées par diffusion de la lumière (méthode statique). Solvant d'étude : DMF/eau = 90/10. Température : 20°C. Le chiffre entre parenthèses représente l'erreur de mesure sur la masse. | | |
| (**) Cette valeur a été déterminée par dosage des fonctions N-hydroxysuccinimide (NHS) en présence d'un excès de NH₄ OH par spectroscopie UV à 260 nm avec e_{NHS} = 7100 l.mole⁻¹.cm⁻¹ pour le NHS dans un mélange eau/DMF/NH₄ OH(0.1M) = 80/10/10. L'erreur de mesure est de 5 %. | | |

Le tableau 3 donne les déplacements chimiques par résonance magnétique nucléaire (RMN) des différents carbones du bipolymère. Les carbones sont identifiés par le numéro tel que décrit dans le schéma ci-dessous :

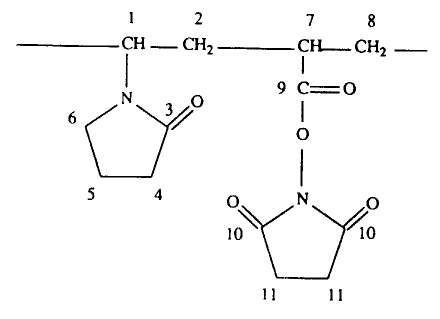

**TABLEAU 3**

| Position du carbone | Déplacement chimique |
|---|---|
| 1 | 48 (raie large) |
| 2 | 34 (raie large) |
| 3 | 175-177,5 (multiplet) |
| 4 | 32,2 (raie large) |
| 5 | 19 (singulet) |
| 6 | 42 (raie large) |
| 7 | 38,5 (raie large) |
| 8 | 34 (raie large) |
| 9 | 163,2 (singulet) |
| 10 | 171 et 170,9 (doublet) |
| 11 | 26,4 et 26,65 (doublet) |
| Solvant : DMF deutéré. Référence interne : Dioxane avec une raie à 67,8 ppm. | |

Les déplacements chimiques sont donnés en partie par million (ppm).

### Couplage de polynucléotides sur des bipolymères :

Les polynucléotides utilisés sont des oligodésoxyribonucléotides de synthèse préparés avec un synthétiseur automatique Applied Biosystems modèle 394 selon le protocole du fabricant.

Ces oligonucléotides possèdent à leur extrémité 5' un bras aliphatique NH2 préparé selon la méthode décrite dans la demande de brevet WO 91/19812.

Par l'intermédiaire des fonctions esters du N-hydroxysuccinimide, il y a réaction directe sur le bras aminé à l'extrémité 5' de l'oligonucléotide pour former une liaison amide.

Le protocole de couplage général est le suivant :
Une solution aqueuse contenant 32 nmoles d'oligonucléotides est versée dans un tube Eppendorf, puis évaporée dans un évaporateur à centrifugation sous vide.

Le résidu sec est repris dans 50 µl de tampon Borate 0.1 M pH = 9.3 puis l'on additionne 400 µl de DMF.
A cette solution sont ajoutés 50 µl d'une solution du bipolymère à 1mg/ml dans le DMF (SDS, référence 340216).

Le tube Eppendorf contenant le milieu réactionnel est agité pendant 16 heures dans un agitateur chauffant pour tubes Eppendorf porté à une température de 37°C.

Le mélange (DMF/eau) est ensuite évaporé à l'évaporateur à centrifugation sous vide.

Le résidu sec est repris dans 50 µl d'eau distillée, afin d'être analysé par électrophorèse capillaire.

### Conditions expérimentales de la séparation par électrophorèse capillaire en solution libre:

- Appareil : 270 A-HT commercialisé par la société APPLIED-BIOSYSTEMS.
- Capillaire de silice d'une longueur de 72 cm (APPLIED-BIOSYSTEMS, réf. 0602-0014).
- Electrolyte de séparation :Tampon Carbonate de Sodium 50 mM, pH = 9,6.
- Voltage appliqué : 20 kilovolts.
- Température: 40°C.
- Injection à l'anode.
- Détection à la cathode, par spectroscopie UV à 260 nm (longueur d'onde d'absorption maximum de l'oligonucléotide).

Le conjugué bipolymère-oligonucléotide est ensuite purifié par chromatographie par perméation de gel (CPG) dans les conditions suivantes:
- Colonne WATERS Ultra Hydrogel 500.
- Tampon phosphate 0.1 M pH = 6.8.
- Détection par spectroscopie UV à 260 nm.
- Débit 0,5 ml/min.

Après purification, le conjugué en solution saline est dialysé pendant une nuit à + 4°C afin d'éliminer le sel. L'eau est éliminée à l'évaporateur à centrifugation sous vide. Le résidu sec est repris dans 1 ml d'eau distillée et stocké au congélateur à la température de - 20°C.

La liste des oligonucléotides utilisés pour le couplage sur un bipolymère est donnée ci-après.

Les caractéristiques des conjugués bipolymère-oligonucléotide sont donnés dans le tableau 4 suivant :

**TABLEAU 4**

| Conjugué | Bipolymère | SEQ ID N° (a) | rendement (b) | Tr (c) | Me (d) | Concentration (e) |
|---|---|---|---|---|---|---|
| CJOA | COPO1 | 2 | 60 | 10,03 | 9,8 | 15,1 |
| CJOB | COPO1 | 3 | 40 | 10,10 | 9,9 | 10,6 |
| CJOC | COPO3 | 2 | 50 | 10,50 | 10,2 | 15,5 |
| CJOD | COPO3 | 4 | 46 | 10,27 | 9,9 | 2,0 |
| CJOE | COPO3 | 5 | 53 | 10,01 | 10,1 | 12,8 |
| CJOF | COPO3 | 6 | 40 | 10,07 | 10,0 | 4,0 |
| CJOG | COPO3 | 1 | 48 | 10,36 | 9,9 | 2,5 |
| CJOH | COPO4 | 2 | 56 | 10,12 | 9,8 | 15,0 |
| CJOI | COPO5 | 2 | 50 | 10,25 | 10,0 | 14,0 |
| (a) Les oligonucléotides ont les séquences suivantes (de 5' vers 3') : | | | | | | |

SEQ ID N° 1 :
TCATCCACCT GGCATTGGAC TGCCATAACC ATGAGTG. . . . . . . . . . . . 37

SEQ ID N° 2 :
TCTAATCCTG TTTGCTCCCC . . . . . . . . . . . . . . . . . . . . 20

SEQ ID N° 3 :
GATGAGCTAT ATGAGAACGG TA . . . . . . . . . . . . . . . . . . . . . . . . . . . 22

SEQ ID N° 4 :
TTAACTTTAC TCCCTTCCTC CCCGCTG . . . . . . . . . . . . . . . . . . . . 27

SEQ ID N° 5 :
TCAATGAGCA AAGGTA . . . . . . . . . . . . . . . . . . . . . . . . . 16

SEQ ID N° 6 :
GGTCCTATCC GGTATTAGAC CCAGTTTCC . . . . . . . . . . . . . . . . . . . 29

Ces oligonucléotides possèdent à leur extrémité 5' un bras aliphatique avec une fonction NH2, préparé selon la méthode décrite dans WO 91/19812.
(b) Le rendement de couplage, calculé par l'intégration des pics en CPG, est le rapport, par chromatographie liquide haute performance (CLHP), de la surface du pic du conjugué sur la somme des surfaces du pic du conjugué et de l'oligonucléotide non couplé. Il est donné en %.
(c) Tr représente le temps de rétention en CPG dans les conditions décrites dans le paragraphe précédent. Il est donné en minutes.
(d) Me représente le temps de rétention donné en minutes dans les conditions de séparation en électrophorèse capillaire décrites dans le paragraphe précédent.
(e) La concentration de 1' oligonucléotide est donnée en picomoles par ml, déterminée par spectrométrie U.V., en mesurant l'absorbance à 260 nm, connaissant le coefficient d'extinction molaire de l'oligonucléotide à cette même longueur d'onde.

### EXEMPLE 2 : Détection d'acides nucléiques d'Escherichia coli par la technique sandwich en utilisant des oligonucléotides couplés des bipolymères comme sonde de capture :

Cet exemple décrit le gain de signal et de sensibilité obtenu par l'usage d'un conjugué oligonucléotide-bipolymère NVP-NAS par rapport à un système où la sonde de capture est constituée du seul oligonucléotide.

Le modèle est la détection par hybridation du groupe taxonomique *Escherichia coli* et *Shigella.* La cible est l'ARN ribosomal 16S (ARNr) de ce groupe. La sonde de capture G3 (SEQ ID N° 4) couplée au bipolymère est complémentaire des nucléotides 440 à 466 de la séquence 16S *d'Escherichia coli* déterminée par Brosius J.M., Palmer L. Kennedy P.J. Noller H.F. (1978) Proc. Natl. Acad. Sci. USA, 75, pages 4801-4805. La sonde de détection G1(SEQ ID N° 5) est constituée de la séquence spécifique du taxon ciblé et le discrimine ainsi des autres espèces bactériennes. Elle est conjuguée à la phosphatase alcaline comme décrit dans le brevet WO 91/19812.

L'hybridation de l'ARNr d'une bactérie-cible a été conduite selon procédé de détection non-radioactif décrit dans le brevet WO 91/19812. Le protocole suivant est effectué automatiquement sur l'automate VIDAS (marque déposée BIOMERIEUX). Les étapes suivantes sont réalisées automatiquement.

La réaction est conduite dans le support appelé SPR (marque de commerce BIOMERIEUX-VITEK) ("Solide Phase Réceptacle") qui est un support conique réalisé à partir d'un copolymère butadiène-styrène vendu sous la dénomination K résine. Les divers réactifs sont disposés dans une barrette à plusieurs puits et les différentes étapes se déroulent dans le SPR qui fait office de pipette. La réaction d'hybridation sandwich décrite dans le protocole ci-dessous se produit sur la paroi interne du cône.

Sur la surface interne du SPR, est fixé passivement soit l'oligonucléotide de capture G3, soit comparativement le conjugué oligonucléotide G3-bipolymère (CJOD, tableau 4). La concentration utilisée dans les deux cas est de 1 ng/µl d'oligonucléotides dans un volume de 300 µl d'une solution de PBS 4x (phosphate de sodium 200 mM pH 7,0, NaCl 600 mM). Après une nuit à température ambiante ou deux heures à 37°C, les cônes sont lavés 2 fois par une solution PBS Tween (PBS 1X, Tween 20: 0,5 % (Merck 822184)), puis séchés sous vide. La barrette contient l'ensemble des réactifs nécessaires à la détection, c'est à dire: 200 µl d'une solution à 0,1 ng/µl du conjugué de détection oligonucléotide G1-phosphatase alcaline, 2 fois 600 µl de solution de lavage PBS Tween et une cuvette de substrat. Dans le premier puits de la barrette, est déposé un volume inférieur ou égal à 100 µl contenant la cible dans un tampon: PBS saumon (phosphate de sodium 150 mM, pH 7,0, NaCl 450 mM + ADN de sperme de saumon 0,1 mg/ml (Sigma D 9156)). Après incubation 40 minutes du cône par le mélange molécule biologique plus sonde de détection, le cône est lavé 2 fois par une solution de PBS Tween. 250 µl de substrat MUP (méthyl-4 umbelliféryl phosphate) en solution dans un tampon diéthanolamine sont aspirés dans le cône, puis relâchés dans une cuvette de lecture. L'appareil mesure le signal fluorescent exprimé en RFU (unités relatives de fluorescence) de la cuvette. Le temps total est de 1 h 30 minutes.

Diverses cibles d'hybridation ont été testées comparativement: soit les bactéries entières (*Escherichia coli* K-12 HB101) en phase de croissance exponentielle, soit l'ARNr purifié (Boehringer Mannheim, référence 206 938), soit une cible synthétique nommée C1 complémentaire des oligonucléotides de capture et de détection et de coordonnées 437-497 correspondant à la séquence :

SEQ ID N° 7 :
TTTCAGCGGG GAGGAAGGGA GTAAAGTTAA TACCTTTGCT CATTGACGTT
ACCCGCAGAA G . . . . . . . . . . . . . . . . . . . . . . . . . . . . . . . . . . . . . . . . 61

Lorsqu'il s'agit de bactéries, l'ARNr est directement extrait dans l'automate par addition de soude jusqu'à une concentration de 0,18N en 1 min suivie d'une neutralisation immédiate par addition d'une quantité équivalente d'acide acétique de même molarité. Une gamme de concentrations de chaque cible a été testée: de 10¹² à 10⁸ copies d'ARNr pur ou de cible synthétique, de 10⁸ à 10⁴ bactéries (dans ce dernier cas en s'assurant que lorsque le nombre d'ARNr en phase exponentielle de croissance est de 10⁴ par bactérie, la gamme de cible ARNr effectivement hybridée est de 10¹² à 10⁸ copies).

Les résultats sont résumés dans le tableau 5.

**TABLEAU 5**

| Cible synthétique C1 ^{a} | | | | | |
|---|---|---|---|---|---|
| Capture | 10¹² | 10¹¹ | 10¹⁰ | 10⁹ | 10⁸ |
| G3 | 11030 | 5641 | 530 | 56 | 9 |
| CJOD | 10238 | 7644 | 1178 | 125 | 45 |
| | | | | | |

| ARN ribosomal ^{a} | | | | | |
|---|---|---|---|---|---|
| Capture | 10¹² | 10¹¹ | 10¹⁰ | 10⁹ | 10⁸ |
| G3 | 1200 | 215 | 36 | 8 | 8 |
| CJOD | 11242 | 6030 | 785 | 89 | 27 |

| Bactéries^{b} | | | | | |
|---|---|---|---|---|---|
| Capture | 10⁸ | 10⁷ | 10⁶ | 10⁵ | 10⁴ |
| G3 | 3040 | 181 | 27 | 10 | 10 |
| CJOD | 11586 | 4326 | 557 | 66 | 17 |

| | | | | | |
|---|---|---|---|---|---|
| a : les niveaux de détection sont exprimés en RFU (unités relatives de fluorescence) pour une quantité de cible exprimée en nombres de copies ou nombre de molécules. | | | | | |
| b : les niveaux de détection sont exprimés en RFU pour une quantité de cible exprimée en nombres de bactéries lysées sachant qu'une bactérie en phase exponentielle de croissance contient en moyenne 10 000 ARN ribosomaux. | | | | | |

L'utilisation du conjugué oligonucléotide-bipolymère améliore nettement l'intensité du signal d'hybridation et la sensibilité sur cible entière (ARNr pur ou extrait de bactéries).

L'amélioration est de l'ordre d'un facteur 100 en sensibilité avec une valeur de bruit de fond de 10-15 RFU. Il ressort de cette expérience qu'il n'y a pas de différence de comportement entre ARNr pur et bactéries lysées à la soude, c'est à dire que la lyse est efficace et que les constituants bactériens autres que la cible n'interfèrent pas dans le test. Dans le cas de la cible synthétique, le gain en sensibilité est moins important.

Du fait de sa taille plus réduite (61 bases), l'encombrement stérique entre la cible et l'oligonucléotide sur le support est diminué, ce qui explique les différences observées entre cette cible synthétique et l'ARN ribosomal.

### EXEMPLE 3 : Détection d'acides nucléiques du complexe tuberculosis par la technique sandwich en utilisant des oligonucléotides couplés à des bipolymères comme sonde de capture :

Cet exemple décrit le gain de signal et de sensibilité obtenu par l'usage d'un conjugué oligonucléotide-bipolymère par rapport à un système où la sonde de capture est constituée du seul oligonucléotide.

Le modèle est la détection par hybridation du groupe taxonomique "complexe *tuberculosis".* La cible est l'ARN ribosomal 16S de ce groupe. La sonde de capture E220 (SEQ ID N 2) couplée au bipolymère est complémentaire des nucléotides 773 à 792 de la séquence 16S d'*Escherichia coli* ou 863-882 de la séquence 16S de Mycobacterium tuberculosis déterminée par Suzuki Y., Nagata A., Ono Y. and Yamada T. (1988), J. Bacteriol., 170, pages 2886-2889. La complémentarité d'E220 aux séquences de ces deux espèces phylogénétiquement éloignées tient au fait que ce motif est conservé chez toutes les eubactéries. La sonde de détection bovis310 est constituée de la séquence spécifique du groupe taxonomique complexe tuberculosis regroupant M. *tuberculosis, M. bovis, M. bovis BCG, M. africanum, M. microti* et le discrimine ainsi des autres espèces bactériennes. Elle possède la séquence :

SEQ ID N° 8 :
ACCACAAGAC ATGCATCCCG TG . . . . . . . . . . . . . . . . . . . . . . 22

Elle est conjuguée à la phosphatase alcaline comme décrit dans l'exemple précédent. On rajoute également en solution un troisième oligonucléotide dTB1 (SEQ ID N° 6) , complémentaire des nucléotides 253 à 281 de *M. tuberculosis* et qui a pour fonction de faciliter l'hybridation de la sonde détection bovis310 en lui étant contigu. Ainsi dans cet exemple les sites de capture et de détection ne sont pas contigus.

L'hybridation des ARNr des souches bactériennes testées a été conduite selon le protocole décrit dans l'exemple 2 à deux exceptions près :
* L'extraction de l'ARNr cible est effectuée selon le protocole de base pour l'extraction de l'ARN des bactéries à Gram positif décrit dans "Current Protocols in Molecular Biology" 1987, Ausubel FM et al., Wiley interscience, New York.
* L'oligonucléotide dTB 1 est ajouté dans la cuvette contenant la sonde de détection (Bovis 310) marquée à la phosphatase alcaline à la concentration de 1 ng/ml.

Le signal obtenu par hybridation sandwich avec l'oligonucléotide de capture E220 et le même oligonucléotide de capture couplé sur le bipolymère (conjugué CJOA, tableau 4) a été comparé. Les résultats sont présentés dans le tableau 6 sur différentes souches de Mycobacterium.

**TABLEAU 6**

| SOUCHES ^{(a)} | | INTENSITE DE DETECTION ^{(b)} | |
|---|---|---|---|
| | | E220 | bipolymère-E220 conjugué CJOA |
| *M. tuberculosis* | A 198 | 91 | 3939 |
| | A 216 (NCTC 7417) | 162 | 7778 |
| *M. bovis* | A 222 | 224 | 7818 |
| *M. bovis BCG* | A 223 | 278 | 7071 |
| *M. intracellulare* | A 227 (ATCC 35764) | 18 | 62 |
| *M. flavescens* | A 234 | 16 | 45 |
| *M. smegtnatis* | A 251 | 11 | 25 |

| | | | |
|---|---|---|---|
| a : la numérotation des souches est une référence interne de la demanderesse. Pour les souches de référence, le code est indiqué entre parenthèses avec l'organisme qui les possède. Les autres souches ont été caractérisées par les méthodes phénotypiques classiques. | | | |
| b : exprimée en unités relatives de fluorescence (RFU). | | | |

Ainsi l'exemple 3 montre que la sonde de détection est spécifique des souches du complexe tuberculosis; en effet, il a été vérifié que les ARNr extraits des autres souches de mycrobactéries étaient bien disponibles pour l'hybridation. De plus, une augmentation de signal est constatée avec le conjugué bipolymère-E220 de capture par rapport à E220 en capture seule. Le facteur d'augmentation moyen du signal est de 35.

### EXEMPLE 4 : Utilisation d'un conjugué oligonucléotide de capture-bipolymère et comparaison à un système où la capture est constituée du seul oligonucléotide :

Le modèle est la détection par hybridation du groupe taxonomique "complexe *tuberculosis".* La cible est l'ARN ribosomal 16S de ce groupe. La sonde de capture dTB1 (SEQ ID N° 6) couplée au bipolymère est complémentaire des nucléotides 253 à 281 de la séquence 16S de *Mycobacterium tuberculosis* déterminée par Suzuki Y., Nagata A., Ono Y. and Yamada T. (1988), J. Bacteriol., 170, pages 2886-2889. La sonde de détection bovis 308 est constituée de la séquence spécifique du groupe taxonomique complexe tuberculosis regroupant *M. tuberculosis, M. bovis, M. bovis BCG, M. africanum, M. microti* et le discrimine ainsi des autres espèces bactériennes. Elle possède la séquence(coordonnées 281-300) :

SEQ ID N° 9 :
ACCACAAGAC ATGCATCCCG . . . . . . . . . . . . . . . . . . . . . . . . . . 20

Elle est conjuguée à la phosphatase alcaline comme décrit précédemment. L'hybridation des ARNr des souches bactériennes testées a été conduite selon le protocole décrit dans l'exemple 2 à l'exception de l'extraction de l'ARNr cible effectuée selon le protocole de base pour l'extraction de l'ARN des bactéries à Gram positif décrit dans "Current Protocols in Molecular Biology" 1987, Ausubel FM et al., Wiley interscience, New York.

Les signaux obtenus par hybridation sandwich avec l'oligonucléotide de capture dTB1 et avec le même oligonucléotide de capture couplé sur le bipolymère (conjugué CJOF, tableau 4) ont été comparés. Les résultats sont présentés dans le tableau 7.

**TABLEAU 7**

| SOUCHES ^{(a)} | Souche n° | INTENSITE DE DETECTION (b) | |
|---|---|---|---|
| | | dTB1 | bipolymère-dTB1 conjugué CJOF |
| *M. tuberculosis* | C001 | 71 | 3905 |
| | C 002 | 135 | 4485 |
| | C 003 | 120 | 4062 |
| | C 120 | 210 | 11437 |
| *M. bovis BCG* | A 221 (ATCC 19210) | 168 | 7818 |
| *M. avium/ intracellulare* | A 227 (ATCC 35764) | 12 | 15 |
| | C 080 | 10 | 9 |
| | C 081 | 11 | 8 |
| *M. flavescens* | C 095 | 11 | 7 |
| *M. smegtnatis* | A 251 | 8 | 8 |

| | | | |
|---|---|---|---|
| a : la numérotation des souches est interne à la demanderesse. Pour les souches déposées, le code est indiqué entre parenthèses avec l'organisme de dépôt. Les autres souches ont été caractérisées par les méthodes phénotypiques classiques. | | | |
| b : exprimée en unités relatives de fluorescence (RFU). | | | |

Ainsi l'exemple 4 montre qu'il se produit une augmentation de signal significative avec le conjugué oligonucléotide-bipolymère en capture et pour un système de séquences différent de l'exemple 3.

Cette augmentation de la sensibilité ne se fait pas au détriment de la spécificité puisque ce système permet de discriminer des espèces proches du genre Mycobacterium.

### EXEMPLE 5 : Utilisation d'un conjugué oligonucléotide de capture-bipolymère et comparaison à un système dans lequel la capture est constituée du seul oligonucléotide. La cible à détecter est constituée d'un fragment de PCR :

Une PCR ("polymerase chain reaction") est effectuée selon le protocole standard tel que décrit dans "PCR PROTOCOLS, A guide to methods and applications" édité par M.A.Innis, D.H. gelfand, J.J. sninsky, T.J. white, Academic Press, sur un plasmide ADN pBR 322 (SIGMA, référence D4904) contenant 4363 paires de bases, en solution dans un tampon Tris 10 mM pH 8,0.Les deux amorces utilisées pour l'amplification sont TEM1 (SEQ ID N° 10) et TEM2 (SEQ ID N° 11):

SEQ ID N° 10 :
CCCCGAAGCG TTTTC . . . . . . . . . . . . . . . . . . . . . . . . . . . . . . . 15

SEQ ID N° 11 :
CGGTATGGTT TGCTGCT . . . . . . . . . . . . . . . . . . . . . . . . . 17

Le fragment de PCR produit est contrôlé sur gel d'acrylamide à 5 %. Il contient 347 paires de bases.

Dans une plaque de microtitration en polystyrène NUNC référence 439454 est déposée 100 µl d'une solution de l'oligonucléotide 1844 (SEQ ID N° 1) à la concentration de 1ng/µl (0,15 µM) dans du tampon PBS 3X (phosphate de sodium 0,15 M, chlorure de sodium 0,45 M pH 7,5). L'incubation s'effectue durant deux heures à la température de 37°C.

En parallèle sur une autre plaque sont déposés 100 µl de conjugué oligonucléotide-bipolymère (CJOG, tableau 4) à la même concentration en oligonucléotide de 1 ng/µl dans le PBS 3X. La plaque est incubée deux heures à 37°C. La plaque est ensuite vidée puis lavée avec 3 x 300 µl de PBS-tween (phosphate de sodium 50 mM, chlorure de sodium 150 µM contenant 0,5 ml/l de Tween 20 référence MERCK 822184).

Le fragment de PCR est dénaturé avant utilisation par mélange de 100 µl de solution de la PCR avec 10 µl de soude 2N. L'ensemble est neutralisé 5 minutes après par l'addition de 10 µl d'acide acétique 2N.

100 µl des dilutions du fragment de PCR dénaturé, dans un tampon PBS-saumon (phosphate de sodium 150 mM, pH 7,0, NaCl 450 mM + ADN de sperme de saumon 0,1 mg/ml (Sigma D 9156)) sont ajoutés dans les puits de la plaque de microtitration. La plaque est incubée une heure à 37°C puis lavée par 3 x 300 µl de PBS-Tween.

Dans chaque puits de la plaque sont ajoutés 100 µl (0,1 ng/µl en oligonucléotides en tampon PBS-saumon) de sonde de détection D1 (SEQ ID N° 12) couplé à la phosphatase alcaline tel que décrit dans l'exemple 2.

SEQ ID N° 12 :
CGCTTTTTTG CACAACATGG GGGATCATG . . . . . . . . . . . . . . . . . . . . . . 29

La plaque est incubée une heure à 37°C puis lavée par 3 x 300 µl de PBS-Tween.

Dans chaque puits de la plaque sont ajoutés 100 µl de substrat PNPP (paranitrophénylphosphate vendu par SIGMA sous la référence N 2765) préparé comme suit : une pastille de 20 mg de substrat est reprise par 10 ml de tampon diethanolamine 1M, 0,5 mM MgCl2, pH 9,8. Après 20 mn de réaction, l'activité enzymatique est bloquée par 100 µl de NaOH 1N.

La lecture de la densité optique est effectué à une longueur d'onde de 402 nm sur un lecteur de plaque AXIA MICROREADER BIOMERIEUX.

Les résultats obtenus sont résumés dans le tableau 8 ci-dessous.

**TABLEAU 8**

| Dilution PCR | 1844 | Bipolymère-1844 CJOG |
|---|---|---|
| 0 | 35 | 70 |
| 1/100.000 | 55 | 280 |
| 1/10.000 | 70 | 490 |
| 1/1.000 | 120 | 864 |
| 1/100 | 335 | 1750 |
| 1/10 | 1200 | 2255 |

Les valeurs obtenues correspondent aux unités de densité optique mesurée sur un appareil AXIA MICROREADER (AXIA marque déposée-BIOMERIEUX).

Il y a bien gain en sensibilité pour détecter un fragment PCR en utilisant comme sonde de capture un conjugué oligonucléotide-bipolymère par rapport à un oligonucléotide.Ce gain en sensibilité ne se fait pas au détriment de la spécificité puisque le signal obtenu pour la dilution de PCR 0, qui correspond à l'absence de cible à détecter, donne un signal plus faible que la dilution la plus basse de 1/100.000.

### EXEMPLE 6 : Utilisation d'un conjugué oligonucléotide de capture-bipolymère dans les immunoessais :

Détection d'alphafoeto protéine (AFP) par protocole sandwich. Comparaison avec la méthode sandwich classique où l'anticorps de capture est adsorbé directement sur le support solide et la méthode utilisant un conjugué bipolymère-oligonucléotide adsorbé sur la phase solide, ledit oligonucléotide étant complémentaire d'un deuxième oligonucléotide couplé de manière covalente à l'anticorps de capture.

Dans une plaque de microtitration en polystyrène NUNC référence 439454 est déposée 100 µl d'une solution de l'anticorps anti(α-foeto protéine) (référence P7H10B7, BIOMERIEUX) dilué à la concentration de 10 µg/ml en tampon carbonate de sodium (Na2CO3 PROLABO référence 27771290-NaHCO3 PROLABO référence 27778293) 50mM, pH 9,6. L'incubation s'effectue durant deux heures à la température de 37°C.

En parallèle sur une autre plaque sont déposés 100 µl de conjugué(CJOB, tableau 4) entre l'oligonucléotide pBP5 (SEQ ID N° 3) couplé à un bipolymère dans du tampon PBS3X. L'incubation s'effectue durant deux heures à la température de 37°C. Après 3 lavages par 300 µl de tampon PBS-tween, 100 µl d'oligonucléotide CpBP5 (SEQ ID N° 13) couplé à l'anticorps anti-AFP (référence P7H10B7 BIOMERIEUX) dilué en tampon PBS-saumon sont ajoutés dans les puits de la plaque de microtitration.

Le couplage entre l'anticorps et l'oligonucléotide CpBP5 est effectué selon la même méthode que décrite pour le couplage oligonucléotide peroxydase dans le brevet WO 91/19812.

SEQ ID N° 13 :
TACCGTTCTC ATATAGCTCA TC. . . . . . . . . . . . . . . . . . . . . . . . . 22

La plaque est incubée une heure à 37°C puis lavée par 3 x 300 µl de PBS-Tween.

Dans chaque puits des deux plaques sont ensuite ajoutés 50 µl d'antigène alphafoeto protéine (AFP) (commercialisé par BEHRING sous la référence OTOD 02/03) dilué en tampon PBS Tween contenant 10 % de sérum de cheval. Pour estimer la sensibilité, les concentrations d'AFP varient de 0 à 100 µg/ml. Sont ensuite additionnés 50 µl d'anticorps anti-AFP (référence P3F11G9, BIOMERIEUX) couplé à la peroxydase selon les méthodes classiques, à la concentration de 0,3 mg/ml en tampon PBS Tween contenant 10% de sérum de cheval. La plaque est incubée une heure à 37°C puis lavée par 3 fois 300 µl de PBS-Tween.

Dans chaque puits de la plaque sont ajoutés 100 µl de substrat OPD (ortho phénylène diamine vendu par SIGMA sous la référence P7288) et préparé comme suit : une pastille de 20 mg de substrat est reprise par 10 ml de tampon citrate pH 4,93(0,055 M acide citrique, 0,1M Na₂ HPO₄ ). Après 30 min de réaction à 37°C, l'activité enzymatique est bloquée par 100 µl d'H₂ SO₄ 1N.

La lecture de la densité optique est effectué à une longueur d'onde de 492 nm sur un lecteur de plaque AXIA MICROREADER.

Les résultats obtenus sont résumés dans le tableau 9.

**TABLEAU 9**

| Concentration d'AFP en µg/ml | Anticorps adsorbé directement | Anticorps fixé par le bipolymère/oligonucléotide |
|---|---|---|
| 0 | 120 | 230 |
| 1 | 125 | 280 |
| 2,5 | 150 | 430 |
| 5 | 150 | 515 |
| 10 | 150 | 905 |
| 50 | 150 | 2500 |
| 100 | 190 | 2500 |

Les valeurs obtenues correspondent aux unités de densité optique mesurée sur l'AXXIA MICROREADER.

Les résultats obtenus montrent une augmentation très forte de la sensibilité du test avec l'emploi du bipolymère-oligonucléotide pour fixer l'anticorps de capture. Une détection de 2,5 µg/ml d'alphafoeto protéine est possible avec le système d'amplification utilisant le bipolymère-oligonucléotide. Le facteur d'augmentation du signal obtenu est supérieur à 10.

### LISTE DES SEQUENCES

SEQ ID N° 1 :
TCATCCACCT GGCATTGGAC TGCCATAACC ATGAGTG

Longueur : 37
Type : acide nucléique
Nombre de brins : simple
Configuration : linéaire
Source : synthèse chimique
Caractéristique additionnelle : bras alkylamine à l'extrémité 5'
Nom d'usage : 1844.

SEQ ID N° 2 :
TCTAATCCTG TTTGCTCCCC

Longueur : 20
Type : acide nucléique
Nombre de brins : simple
Configuration : linéaire
Source : synthèse chimique
Caractéristique additionnelle : bras alkylamine à l'extrémité 5'
Nom d'usage : E220.

SEQ ID N° 3 :
GATGAGCTAT ATGAGAACGG TA

Longueur : 22
Type : acide nucléique
Nombre de brins : simple
Configuration : linéaire
Source : synthèse chimique
Caractéristique additionnelle : bras alkylamine à l'extrémité 5'
Nom d'usage : pBP5.

SEQ ID N° 4 :
TTAACTTTAC TCCCTTCCTC CCCGCTG

Longueur : 27
Type : acide nucléique
Nombre de brins : simple
Configuration : linéaire
Source : synthèse chimique
Caractéristique additionnelle : bras alkylamine à l'extrémité 5'
Nom d'usage : G3.

SEQ ID N° 5 :
TCAATGAGCA AAGGTA

Longueur : 16
Type : acide nucléique
Nombre de brins : simple
Configuration : linéaire
Source : synthèse chimique
Caractéristique additionnelle : bras alkylamine à l'extrémité 5'
Nom d'usage : G1.

SEQ ID N° 6 :
GGTCCTATCC GGTATTAGAC CCAGTTTCC

Longueur: 29
Type : acide nucléique
Nombre de brins : simple
Configuration : linéaire
Source : synthèse chimique
Caractéristique additionnelle : bras alkylamine à l'extrémité 5'
Nom d'usage : DTB1.

SEQ ID N° 7 :
TTTCAGCGGG GAGGAAGGGA GTAAAGTTAA TACCTTTGCT CATTGACGTT
ACCCGCAGAA G

Longueur : 61
Type : acide nucléique
Nombre de brins : simple
Configuration : linéaire
Source : synthèse chimique
Nom d'usage: C1.

SEQ ID N° 8:
ACCACAAGAC ATGCATCCCG TG

Longueur: 22
Type : acide nucléique
Nombre de brins : simple
Configuration : linéaire
Source : synthèse chimique
Caractéristique additionnelle : bras alkylamine à l'extrémité 5'
Nom d'usage : bovis310.

SEQ ID N° 9 :
ACCACAAGAC ATGCATCCCG

Longueur: 20
Type : acide nucléique
Nombre de brins : simple
Configuration : linéaire
Source : synthèse chimique
Caractéristique additionnelle : bras alkylamine à l'extrémité 5'
Nom d'usage : bovis308.

SEQ ID N° 10 :
CCCCGAAGCG TTTTC

Longueur : 15
Type : acide nucléique
Nombre de brins : simple
Configuration : linéaire
Source : synthèse chimique
Nom d'usage : TEM1.

SEQ ID N° 11 :
CGGTATGGTT TGCTGCT

Longueur : 17
Type : acide nucléique
Nombre de brins : simple
Configuration : linéaire
Source : synthèse chimique
Nom d'usage : TEM2.

SEQ ID N° 12 :
CGCTTTTTTG CACAACATGG GGGATCATG

Longueur : 29
Type : acide nucléique
Nombre de brins : simple
Configuration : linéaire
Source : synthèse chimique
Caractéristique additionnelle : bras alkylamine à l'extrémité 5'
Nom d'usage : D1.

SEQ ID N° 13 :
TACCGTTCTC ATATAGCTCA TC

Longueur : 22
Type : acide nucléique
Nombre de brins : simple
Configuration : linéaire
Source : synthèse chimique
Caractéristique additionnelle : bras alkylamine à l'extrémité 5'
Nom d'usage : CpBP5.

## Revendications

1. Dispositif de capture d'une molécule cible, dans le but de la détecter et/ou de la doser, par mise en contact dudit dispositif avec un véhicule liquide contenant ladite molécule cible en solution, ledit dispositif comprenant un support solide sur lequel est immobilisé un ligand capable de former un complexe de type ligand/anti-ligand, ledit ligand étant présent sous la forme d'un conjugué résultant du couplage covalent d'un polymère avec une pluralité de molécules dudit ligand, caractérisé par le fait que ledit polymère est un copolymère de N-vinyl pyrrolidone et que ledit conjugué, qui est soluble dans ledit véhicule liquide, est immobilisé sur le support solide par adsorption.

2. Dispositif selon la revendication 1, caractérisé par le fait que le copolymère provient de la copolymérisation d'un monomère de N-vinyl pyrrolidone et d'un deuxième monomère permettant l'établissement d'un couplage covalent entre le ligand et le copolymère.

3. Dispositif selon la revendication 2, caractérisé par le fait que le deuxième monomère porte une fonction réactive ou une fonction activable permettant la formation d'une liaison covalente entre le copolymère et le ligand, ladite fonction réactive étant choisie parmi les fonctions aldéhyde, époxy, halogénoalkyle, amine primaire, thiol, maléimide ou ester, en particulier ester de N-hydroxysuccinimide, et ladite fonction activable étant choisie parmi les fonctions carboxyle et hydroxyle.

4. Dispositif selon la revendication 2 ou 3, caractérisé par le fait que le second monomère est le N-acryloxysuccinimide.

5. Dispositif selon l'une quelconque des revendications précédentes, caractérisé par le fait que ledit copolymère contient de 25 à 70 %, en motifs, de motifs de N-vinyl pyrrolidone.

6. Dispositif selon l'une quelconque des revendications précédentes, caractérisé par le fait que ledit anti-ligand constitue la molécule cible.

7. Dispositif selon la revendication 6, caractérisé par le fait que ledit complexe est choisi parmi les complexes antigène/anticorps, anticorps/haptène, hormone/récepteur, chélatant/molécule chélatée, les hybrides polynucléotide/polynucléotide, polynucléotide/acide nucléique.

8. Dispositif selon l'une quelconque des revendications précédentes, caractérisé par le fait qu'il comprend en outre un réactif bifonctionnel comprenant un groupement anti-ligand, permettant la formation d'un complexe avec le ligand, ledit groupement anti-ligand étant lié avec un groupement partenaire de la cible, capable de former un complexe partenaire/cible.

9. Dispositif selon la revendication 8, caractérisé par le fait que le groupement anti-ligand est capable de former avec le ligand un complexe, ledit complexe étant choisi parmi les complexes antigène/anticorps, anticorps/haptène, hormone/récepteur, hybride polynucléotide/polynucléotide, hybride polynucléotide/acide nucléique, chélatant/molécule chélatée, biotine/streptadivine et lectine/sucre.

10. Dispositif selon l'une quelconque des revendications 1 à 9, caractérisé par le fait que ledit ligand est un polynucléotide contenant de 5 à 100 nucléotides.

11. Procédé de capture de molécules cibles, dans lequel on met en contact une solution de la molécule cible avec un dispositif comprenant un support solide sur lequel est immobilisé un ligand, dans des conditions permettant la formation d'un complexe de type ligand/anti-ligand, caractérisé par le fait que ledit dispositif est tel que défini dans l'une quelconque des revendications précédentes.

## Patentansprüche

1. Vorrichtung zum Fangen eines Zielmoleküls zum Zweck des Nachweises und/oder der Mengenbestimmung durch Inkontaktbringen der Vorrichtung mit einem flüssigen Träger, der das Zielmolekül in Lösung enthält, wobei die Vorrichtung einen festen Träger umfaßt, auf dem ein Ligand immobilisiert ist, der einen Komplex vom Typ Ligand/Anti-Ligand bilden kann, wobei der Ligand in Form eines aus der kovalenten Kupplung eines Polymeren mit einer Vielzahl von Molekülen des Liganden resultierenden Konjugats vorhanden ist, dadurch gekennzeichnet, daß das Polymer ein N-Vinylpyrrolidoncopolymer ist und das Konjugat, das in dem flüssigen Träger löslich ist, auf dem festen Träger über Adsorption immobilisiert ist.

2. Vorrichtung gemäß Anspruch 1, dadurch gekennzeichnet, daß das Copolymer aus der Copolymerisation eines N-Vinylpyrrolidonmonomeren mit einem zweiten Monomeren hervorgeht, welches die Ausbildung einer kovalenten Kupplung zwischen dem Liganden und dem Copolymer gestattet.

3. Vorrichtung gemäß Anspruch 2, dadurch gekennzeichnet, daß das zweite Monomer eine reaktive Funktion oder eine aktivierbare Funktion trägt, die die Bildung einer kovalenten Bindung zwischen dem Copolymeren und dem Ligand gestattet, wobei die reaktive Funktion unter Aldehyd-, Epoxy-, Halogenalkyl-, primären Amino-, Thiol-, Maleimid- oder Esterfunktionen, insbesondere dem N-Hydroxysuccinimidester ausgewählt ist und die aktivierbare Funktion unter Carboxyl- und Hydroxylfunktionen ausgewählt ist.

4. Vorrichtung gemäß Anspruch 2 oder 3, dadurch gekennzeichnet, daß das zweite Monomer N-Acryloxysuccinimid ist.

5. Vorrichtung gemäß einem der vorstehenden Ansprüche, dadurch gekennzeichnet, daß das Copolymer 25 bis 70 %, bezogen auf Einheiten, N-Vinylpyrrolidon-Einheiten enthält.

6. Vorrichtung gemäß einem der vorstehenden Ansprüche, dadurch gekennzeichnet, daß der Anti-Ligand das Zielmolekül darstellt.

7. Vorrichtung gemäß Anspruch 6, dadurch gekennzeichnet, daß der Komplex ausgewählt ist unter den Komplexen von Antigen/Antikörper, Antikörper/Hapten, Hormon/Rezeptor, Chelatbildner/chelatiertes Molekül, Polynukleotid/Polynukleotid-Hybriden, Polynukleotid/Nukleinsäure-Hybriden.

8. Vorrichtung gemäß einem der vorstehenden Ansprüche, dadurch gekennzeichnet, daß sie zusätzlich ein bifunktionelles Reagenz enthält, welche eine Anti-Liganden-Gruppe aufweist, die die Bildung eines Komplexes mit dem Liganden gestattet, wobei die Anti-Liganden-Gruppe sich an eine Partnergruppe des Ziels binden kann, befähigt zur Bildung eines Partner/Ziel-Komplexes.

9. Vorrichtung gemäß Anspruch 8, dadurch gekennzeichnet, daß die Anti-Liganden-Gruppe mit dem Ligand einen Komplex bilden kann, wobei der Komplex ausgewählt ist unter den Komplexen von Antigen/Antikörper, Antikörper/Hapten, Hormon/Rezeptor, Polynukleotid/Polynukleotid-Hybrid, Polynukleotid/Nukleinsäure-Hybrid, Chelatbildner/chelatiertes Molekül, Biotin/Streptadivin und Lectin/Zukker.

10. Vorrichtung gemäß einem der Ansprüche 1 bis 9, dadurch gekennzeichnet, daß der Ligand ein Polynukleotid, enthaltend 5 bis 100 Nukleotide, darstellt.

11. Verfahren zum Einfangen eines Zielmoleküls bei dem man eine Lösung des Zielmoleküls unter Bedingungen, welche die Bildung eines Komplexes vom Typ Ligand/Anti-Ligand gestatten, mit einer Vorrichtung in Kontakt bringt, umfassend einen festen Träger, auf dem ein Ligand immobilisiert ist, dadurch gekennzeichnet, daß die Vorrichtung gemäß einem der vorstehenden Ansprüche definiert ist.

## Claims

1. Device for capturing a target molecule for the purpose of detecting it and/or assaying it, by bringing the said device into contact with a liquid vehicle containing the said target molecule in solution, the said device comprising a solid support on which is immobilized a ligand capable of forming a complex of ligand/antiligand type, the said ligand being present in the form of a conjugate resulting from the covalent coupling of a polymer with a plurality of molecules of the said ligand, characterized by the fact that the said polymer is an N-vinylpyrrolidone copolymer, and that the said conjugate, which is soluble in the said liquid vehicle, is immobilized on the solid support by adsorption.

2. Device according to Claim 1, characterized by the fact that the copolymer results from the copolymerization of an N-vinylpyrrolidone monomer and a second monomer permitting the establishment of a covalent coupling between the ligand and the copolymer.

3. Device according to Claim 2, characterized by the fact that the second monomer carries a reactive functional group or an activable functional group permitting the formation of a covalent bond between the copolymer and the ligand, the said reactive functional group being chosen from aldehyde, epoxy, haloalkyl, primary amine, thiol, maleimide and ester functional groups, in particular N-hydroxysuccinimide ester, and the said activable functional group being chosen from carboxyl and hydroxyl functional groups.

4. Device according to Claim 2 or 3, characterized by the fact that the second monomer is N-acryloxysuccinimide.

5. Device according to any one of the preceding claims, characterized by the fact that the said copolymer contains from 25 to 70 %, in units, of N-vinylpyrrolidone units.

6. Device according to any one of the preceding claims, characterized by the fact that the said anti-ligand constitutes the target molecule.

7. Device according to Claim 6, characterized by the fact that the said complex is chosen from the antigen/antibody, antibody/hapten, hormone/receptor or chelator/chelated molecule complexes and the polynucleotide/polynucleotide or polynucleotide/nucleic acid hybrids.

8. Device according to any one of the preceding claims, characterized by the fact that it comprises, in addition, a bifunctional reagent comprising an anti-ligand group, permitting the formation of a complex with the ligand, the said anti-ligand group being bonded with a partner group for the target, capable of forming a partner/target complex.

9. Device according to Claim 8, characterized by the fact that the anti-ligand group is capable of forming, with the ligand, a complex, the said complex being chosen from the antigen/antibody, antibody/hapten, hormone/ receptor, polynucleotide/polynucleotide hybrid, polynucleotide/nucleic acid hybrid, chelator/chelated molecule, biotin/streptadivin and lectin/sugar complexes.

10. Device according to any one of Claims 1 to 9, characterized by the fact that the said ligand is a polynucleotide containing 5 to 100 nucleotides.

11. Process for capturing target molecules, in which a solution of the target molecule is brought into contact with a device comprising a solid support on which is immobilized a ligand, under conditions permitting the formation of a ligand/anti-ligand type complex, characterized by the fact that the said device is as defined in any one of the preceding claims.
